Europäisches Patentamt

**European Patent Office**

**Office européen des brevets**

(19)

(11) Publication number: **0 239 039**
**A2**

(12) # EUROPEAN PATENT APPLICATION

(21) Application number: **87104163.8**

(22) Date of filing: **20.03.87**

(51) Int. Cl.⁴: **A 61 D 7/00**

(30) Priority: **24.03.86 US 843334**

(43) Date of publication of application: **30.09.87**
**Bulletin 87/40**

(84) Designated Contracting States: **AT BE CH DE ES FR GB
GR IT LI LU NL SE**

(71) Applicant: **SYNTEX (U.S.A.) INC., 3401 Hillview Avenue,
Palo Alto, California 94304 (US)**

(72) Inventor: **Blackhall, William J., 49 Renway Avenue
Lugarno, N.S.W. 2210 Sydney (AU)**

(74) Representative: **Gudel, Diether, Dr. et al, Patentanwälte
Dr. V. Schmied-Kowarzik Dipl.-Ing. G. Dannenberg Dr. P.
Weinhold Dr. D. Gudel Dipl.-Ing. S. Schubert Dr. P. Barz
Grosse Eschenheimer Strasse 39, D-6000 Frankfurt am
Main 1 (DE)**

(54) **Intraruminal controlled release device.**

(57) An intraruminal controlled-release device has a dispenser component (optionally containing a therapeutic agent), which may be of a type known to the art, joined by a flexible connection to a separate weight component. The dispenser is of lower density than the average rumen content, and thus floats at least partially within the ruminal fluid, while the weight prevents regurgitation. The flexible connection is preferably short to minimize the chance of entanglement with rumen contents.

-1-

INTRARUMINAL CONTROLLED-RELEASE DEVICE

FIELD OF THE INVENTION

This invention relates to a controlled-release device for administration of therapeutic agents to ruminants.

BACKGROUND OF THE INVENTION

There currently are available several chemical compounds, collectively referred to herein as "therapeutic agents", which exhibit a beneficial affect upon ruminant animals. For example, several compounds are known which improve feed efficiency when administered to ruminants. Other compounds are effective in promoting growth and improving the yield of meat utilized for human consumption. Still other compounds are effective in the therapeutic treatment and prophylactic control of ruminant diseases such as parasitic infestation. Many of these useful agents are effective only when administered via the rumen of the animal.

2767Y                                                    25430-FF

Examples of such agents include water soluble anthelmintics such as morantel, pyrantel, tetramisole, levamisole, butamisole, nitramisole and diethyl carbamazine and salts thereof, and salts of nitroxynil such as the N-ethyl glucamine salt; more suitably morantel, pyrantel, tetramisole, levamisole, or a salt thereof such as the hydrochloride. Piperazine and salts thereof may also be used. Preferred examples of water soluble anthelmintics include morantel or a salt thereof, such as salts with organic acids, e.g. citrate and tartrate, and levamisole or a salt thereof such as the hydrochloride. Sparingly soluble salts of the above water soluble anthelmintics may also be used, especially the pamoates.

Water insoluble and sparingly soluble anthelmintics may also be used. These suitably include oxfendazole, fenbendaxole, albendazole, albendazole sulfoxide [5-n-propylsulfinyl-2-carbomethoxybenzimidazole], oxibendazole, cambendazole, parbendazole, thiabendazole, febantel, thiophanate, diamphenethide, rafoxanide, flubendazole and mebendazole.

Also suitably any of the anthelmintics described in Offenlegungsschrift No. 28 36 690 such as 1-[2-(4-chlorobenzylideneamino)-5-n-propylthiophenyl]-3-methoxycarbonyl-S-methylisothiourea may be used.

Other suitable agents include insecticides, bloat inhibitors (such as surfactants), coccidiostats, vitamins, mineral supplements (such as copper, selenium, cobalt or magnesium), growth promoters, and agents useful for the control of trypanosomiasis, babesiasis and diseases related thereto. Combinations of two or more of these therapeutic agents, such as an anthelmintic and a growth promoter, two anthelmintics (e.g. oxfendazole + rafoxanide), etc., may of course be used.

Many of these agents routinely are administered by daily dosing in the form of feed additives, licks, water additives and the like. Because some animals are range fed over a rather long period of time, it is impossible to administer drugs which require daily administration or delivery via feed additives or the like. Moreover, daily administration of drugs by injection to feed lot animals is uneconomical.

Slow release formulations designed to release an effective amount of active ingredient over a prolonged period of time are known in the art. However, many problems have been encountered in the development of effective means for economically administering such controlled release formulations to the rumen of domesticated ruminant animals. This is due to a number of factors. If the delivery device is too small in size and weight, it will not remain in the rumen sufficiently long to be effective, nor will it contain a sufficient quantity of active ingredient to achieve the desired sustained release effect. If the device is too large, administration to the animal is difficult or impossible. Moreover, foreign devices placed in the rumen are prone to expulsion by regurgitation or by passage into the intestine. Most important, however, is the fact that the delivery device must not permit excessive amounts of formulated drug to be administered to the animal, since excessive doses of certain drugs may be lethal.

A number of ruminant devices have been designed in an effort to overcome these problems. These devices are of two principal types: those which are retained in the rumen because of their shape (variable-geometry devices), and those which are retained in the rumen because of their weight.

Of the first type, Laby, in U.S. Patent No. 3,844,285, describes an expandable device which allegedly is orally administered and then once in the rumen is adapted to physically change into an altered configuration which will prevent or at least hinder regurgitation of the device. Similarly, a spring-loaded device is described by Laby in U.S. Patent No. 4,251,506. This device is equipped with expandable plastic strips which are said to be effective in retaining the device in the rumen following oral administration.

Similar devices are shown by Laby et al. in PCT Published Application No. WO 82/00094 and British Published Application No. 2 115 073, and by Simpson et al. in U.S. Patent No. 4,416,659. Griffin et al., in U.S. Patent No. 4,268,497, describe a rolled erodible sheet device designed to unroll in the rumen to a planar form which aids retention in the rumen; while Guerrero et al., in U.S. Patent No. 4,326,522, describe a mesh-covered bolus where the configuration aids retention in the rumen.

Of the second type, Simpson, in European Published Application No. 25699, describes a coated hollow steel cylinder to be filled with a formulated drug core. The inner wall of the cylinder, which is open at both ends, has retaining means to prevent premature expulsion of the core. Dresback, in British Published Application No. 2 020 181, describes a bolus having a porous wall with pores containing a hydrogel in contact with a reservoir containing a chemical and a water-soluble excipient or detergent. The bolus is weighted (either by use of a porous metal sleeve or by the incorporation of e.g. metal shot in the bolus material). Lucas, in U.S. Patent No. 4,505,711, describes a device of cotton-reel shape, in which the device and/or its load are weighted to a density of at least $2 \text{ g} \cdot \text{mL}^{-1}$. Holloway, in U.S. Patent No. 4,381,780, describes another form of weighted

2767Y                                                25430-FF

bolus.  These weighted devices may all be described as being "unitary", in the sense that the weight component. and the dispensing component are not separate.

Devices having some of the characteristics of both types are described by Laby in Australian Patent No. 470538 (which shows two identical body portions of magnesium alloy pivotally connected by an insulating hinge, e.g. of polymeric material) and in British Published Application No. 2 138 288 (where two identical body portions of magnesium alloy are pivotally connected by a conductive polymeric material such as carbon-loaded rubber).  These devices are classed by the inventor as variable-geometry devices, though they are made of metal, and therefore somewhat  dense.

The disclosures of each of these patents and applications is incorporated herein by reference.

The prior art devices suffer from various disadvantages.  In general, the variable-geometry devices of Laby, Laby et al., and Simpson et al. are subject to mechanical failure of the "wings" which prevent regurgitation, while the Griffin et al. device may "ball-up" to a small size again, possibly permitting regurgitation.  The weighted devices lodge at the bottom of the rumen, where circulation of ruminal contents is low, thereby possibly limiting dissolution of the therapeutic agent, or, less desirably, within the reticulum, where the therapeutic agent dissolved from the device may rapidly pass into the omasum rather than remaining for a longer time in the rumen.

It would be desirable to provide an intraruminal controlled-release device which achieves satisfactory distribution of the therapeutic agent contained therein to the rumen.

## SUMMARY OF THE INVENTION

This invention relates to an intraruminal controlled-release device for dispensing a therapeutic agent to the rumen of a ruminant animal, which device comprises: dispensing means for dispensing the therapeutic agent, the dispensing means (either as such or together with the therapeutic agent) being of lower density than the ruminal contents of the animal; weight means separate from the dispensing means; and flexible connecting means for connecting the dispensing means to the weight means; wherein the weight means is of sufficient size and density to locate at the bottom of the rumen and, with the connecting means, hold the dispensing means at least partially within the ruminal contents. The connecting means preferably is short, but sufficiently long to allow the orientation of the dispensing means to be independent of that of the weight means. This invention also relates to the device described immediately above which is loaded with a therapeutic agent.

## BRIEF DESCRIPTION OF THE DRAWING

Figure 1 shows, conceptually, a cross-section of a rumen, with the device of this invention therein.

Figure 2 shows a preferred device in cross-section within a rumen.

Figure 3 shows a configuration for the device when it is prepared for insertion into an animal via the esophagus.

## DETAILED DESCRIPTION OF THE PREFERRED EMBODIMENT

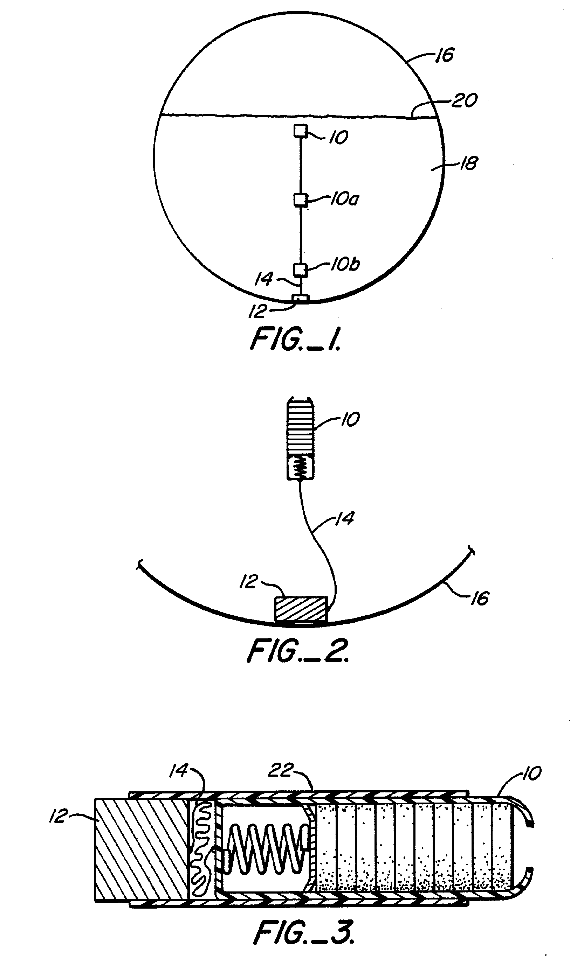

As shown in Figure 1, the intraruminal controlled-release device of this invention comprises three components. The first component is a dispensing means 10 for dispensing the therapeutic agent; the

2767Y                                                          25430-FF

dispensing means (either as such or together with the therapeutic agent, preferably both) being of lower density than the ruminal contents of the animal. The dispensing means thus floats at least partially, preferably completely, within the ruminal fluid, allowing maximum uniformity of release of the therapeutic agent.

The dispensing means may be loaded with a therapeutic agent, and this invention includes the device both unloaded and when it is loaded with a therapeutic agent. The therapeutic agent may be one of those exemplified in the prior art as desirably administered to ruminant animals, especially those administered in a controlled, non-single dose fashion, either by continuous release over some period, or by pulsed release during that period, as discussed in, e.g., U.S. Patent No. 4,251,506. Preferred therapeutic agents comprise anthelmintics, such as benzimidazole anthelmintics, e.g. oxfendazole or oxfendazole + rafoxanide, etc.

The dispensing means 10 may be of any type suitable for the release of the therapeutic agent, and suitable such dispensing means are exemplified in the prior art, such as in the references discussed in the BACKGROUND OF THE INVENTION section of this application. The dispensing means, though, will not require the variable-geometry features, such as the "wings", or the weight of the prior art disclosures, but may be optimized simply for delivery of the therapeutic agent, and choice of a suitable dispensing means for a desired therapeutic agent will be within the skill of a person of ordinary skill in the art having regard to this disclosure, without undue experimentation. A preferred dispensing means is the spring-loaded dispenser shown in Figure 1 of U.S. Patent No. 4,251,506 (although without the "wings" of that device). The dispenser there shown comprises a tube, partially open at one end. A spring, acting

2767Y                                                    25430-FF

between the other (closed) end and a plug within the tube urges the plug towards the partially open end. The plug, in turn, urges a cast mass of an erodible composition containing the therapeutic agent towards the partially open end, where it is gradually eroded by the action of the animal's ruminal fluid, releasing the therapeutic agent into the rumen. Such a dispensing means is depicted in Figures 2 and 3 of this application, though no limitation as to suitable dispensing means for use in this invention is intended by this depiction.

As stated previously, the dispensing means, either as such or together with the therapeutic agent (or preferably both), should be of lower density than the ruminal contents of the animal (which typically has a density of about 1.05 $g \cdot mL^{-1}$), so that it floats at least partially, preferably completely, within the ruminal fluid. Since suitable dispensing means may typically be made from polymers such as polyolefins (polyethylene, polypropylene, etc.) and other easily-molded polymers, and the therapeutic agent may typically be dispersed in a wax or similar material, both the dispensing means and the therapeutic agent then being generally of density less than 1 $g \cdot mL^{-1}$, and there may be air spaces within the dispensing means to provide added buoyancy, it is not expected that any difficulty will be encountered in choice of a suitable dispensing means.

It is within the scope of this invention for the dispensing means to be bioerodible to permit its disintegration after the expected dispensing lifetime (complete release of the therapeutic agent). However, it is important that any bioerodibility should be such that the dispensing means not disintegrate during the expected dispensing lifetime, to prevent excessively rapid release of the therapeutic agent.

The weight means 12 will be of sufficient size and density to locate at the bottom of the rumen and, with the connecting means, hold the dispensing means at least partially within the ruminal contents, and may be of any material suitable for that purpose. Desirably, the weight means will be of the maximum density conveniently possible (to minimize its required size) and, typically, it will comprise a metal or metal alloy (as, e.g., iron-containing alloys such as mild or stainless steel); which, if not itself sufficiently inert to ruminal fluids for the expected dispensing lifetime of the device, will preferably be coated with an inert material, for example, polymers, e.g. polyolefins (polyethylene, polypropylene, poly(fluoroolefins) such as Teflon*, etc.), polyamides (nylon, etc.), polyesters, epoxies, and the like. A suitable coated weight means could thus comprise an iron alloy coated with an epoxy. A typical density for the weight means will thus be approximately $6 - 10$ $g \cdot mL^{-1}$.

The device density, i.e. the sum of the masses of the components (dispensing means + weight means + connecting means) divided by the sum of the volumes of the components, should be at least 1.5, preferably at least 2, more preferably at least 2.2, $g \cdot mL^{-1}$, to prevent regurgitation and assure retention in the rumen. A straightforward calculation, exemplified later, will determine the necessary size of the weight means for a given dispensing means and desired device density. Similarly, the loaded device density, i.e. the device density when the dispensing means is loaded with the therapeutic agent, as prior to administration to the animal, should also be as described immediately above. For the reasons explained before for the density of the dispensing means, this should be readily achievable.

The connecting means 14 will be a flexible means for connecting the dispensing means 10 and the separate weight means 12. It should be of sufficient strength to hold the dispensing means and weight means together under the conditions obtaining in the rumen, while being sufficiently flexible to permit independent relative orientation of the dispensing means and weight means, as stated previously. It will preferably be a filament (by which term is included a strand, wire, chain, strip, string, or thread), e.g. of polymeric material, such as one of the materials described above for coating the weight means or forming the dispensing means, such as a nylon string, a filament of the material of the dispensing means (e.g. polyethylene) co-molded therewith and extending therefrom, or a coated wire, etc.

It is also within the scope of this invention, and a preferred feature thereof, that the dispensing means shall be separable from the weight means after complete release of the therapeutic agent to permit regurgitation or passage of the dispensing means. This may be achieved, for example, by the connecting means being of such strength and resistance to ruminal fluid that, sometime after complete release of the therapeutic agent, the connecting means may break; or by the connection of the connecting means to the dispensing means being such that it is broken when complete release of the therapeutic agent has occurred (as, e.g., by connecting the connecting means to the dispensing means via the therapeutic agent, so that complete dissolution of the therapeutic agent releases the connecting means from the dispensing means).

The length of the connecting means 14 should be such that, when the weight means locates on the bottom of the rumen, the dispensing means floats at least partially, and preferably completely, within the ruminal contents. Figure 1 illustrates a range of possible lengths for the connecting means 14 within that constraint. In the

2767Y

Figure, which represents a cross-sectional view of a rumen, the rumen 16 is partly filled with contents 18, typically a mixture of ruminal fluid and partially digested food, to the contents top surface 20. The connecting means 14 may be of such a length that the dispensing means 10 floats near the top of the contents (10), midway within the rumen (10a), or relatively near the bottom of the rumen (10b). The length chosen for the connecting means 14 will also depend on the size and shape of the dispensing means 10 and weight means 12.

In Figure 2, dispensing means 10 is shown as being of the type illustrated in U.S. Patent No. 4,251,506 (though without the "wings"), attached at the spring end thereof to the connecting means 14. The weight means 12 is attached at the center of an end thereof to the other end of the connecting means 14. It is preferred, to minimize possible entanglement of the connecting means with relatively solid ruminal contents, that the connecting means be relatively short, provided that it is sufficiently long that the dispensing means may adopt an orientation in the rumen (here shown as vertical with the dispensing opening uppermost) which is independent of the orientation of the weight means.

The size and shape of the dispensing means and weight means are generally dictated by their function and the ability of the device to be administered to an animal (a factor which imposes maximum length and cross-sectional dimensions on the device). Typically, the dispensing means and weight means will each be cylindrical, i.e. of a constant cross-section (the same for both weight and dispensing means) along their length, preferably in the form of a circular cylinder, as a circular cross-section offers the maximum volume for a given external dimension. A cylindrical configuration is typical of prior art devices. The connecting means may be attached to the dispensing means and weight means at any suitable point on each, preferably in such a way that the orientation of the dispensing means may be independent of the orientation of the weight means. Typically, the connecting means may be connected to the center of the cross-section of the dispensing means (e.g., at the spring end of the device of U.S. Patent No. 4,251,506) or at its axial mid-point (especially for a device open at both ends); and be connected in a similar position to the weight means. For example, the connecting means may comprise a filament molded as a "tail" from the dispensing means at the center of one end, and connect to the weight means by passing through an axial hole therein, being fastened by a crosspiece or arrowhead on the filament near its end which, when the filament end is passed through the hole in the weight means, prevents easy withdrawal thereof.

A suitable minimum length of the connecting means would be about the diameter of the device (as used herein, the term "diameter" will refer to the greatest radial dimension of the device, even if it is not of circular cross-section) if the connecting means is connected to the end of the axis of each of the dispensing means and the weight means, or rather less (e.g., half a diameter) if the connecting means is connected to the midpoint of one or both. A suitable maximum length would be dependent on the size and shape of the dispensing means and weight means, and on the position of the connection, as well as the size of the animal to which the device is to be administered, but a preferable maximum length would be less than five diameters, more preferably less than three diameters. A typical suitable length for the connecting means will be about two diameters.

The device should be sized to be capable of introduction into the rumen through the esophagus, and typical maximum sizes would be:

for cattle, 150-180mm in length, 30-40mm in diameter;

for calves, 100-120mm in length, 20-30mm in diameter;

for sheep/goats, 60-110mm in length, 15-20mm in diameter.

Devices smaller than these maximum sizes may, of course, be employed if desired, depending on the size of the animal and the therapeutic agent (which at least partially determines the size of the dispensing means). Typically, however, devices near (e.g. within 30% of) the maximum sizes given above will be used, as larger devices contain greater proportions of therapeutic agent and it is generally desired to minimize the number of administrations of the device.

The device of the present invention may be inserted into the rumen, generally through the esophagus, by any suitable means, such as a balling tube, balling gun, or the like, as are well-known in the art. A device such as that shown in Figure 8 of U.S. Patent No. 4,416,659 may also be used.

Figure 3 shows a configuration for the device when it is prepared for insertion into an animal via the esophagus. In this Figure, the device, in which the connecting means 14 is connected to the ends of the dispensing means 10 and weight means 12 at their axes, has been encased within a tube 22 of a material readily degraded within the rumen, such as a kraft paper tube. The tube 22 may also be created by wrapping the device with a tape, also of kraft paper or the like with a non-toxic, preferably water-soluble, adhesive. In this manner, the possibility of breakage of the connecting means during administration is minimized, and handling of the device is rendered easier. Once the encased device enters the rumen of the animal, the tube rapidly degrades to release the device, which then functions as previously described.

### Example 1

An exemplary device for use in sheep or goats may be sized as follows:

For a device of total length 100 mm and diameter 17 mm, and having a final density of 2 $g \cdot mL^{-1}$; the volume of the device is 22.7 mL, and its mass 45.4 g.

Plastic components for such a device have a mass of approximately 8.8 g, and the therapeutic agent payload is approximately 8.9 g in mass (45 mm length, allowing room for plug, spring, etc.).

A weight means of mass 27.7 g is required; which represents a length of 15 mm of iron/steel at the same 17 . mm diameter as the dispensing means.

A suitable length for the connecting means is between about 15 and 50 mm, preferably about 35 mm.

### Example 2

An exemplary device for use in calves may be sized as follows:

For a device of total length 120 mm and diameter 25 mm, and having a final density of 2.05 $g \cdot mL^{-1}$; the volume of the device is 58.9 mL, and its mass 121 g.

Plastic components for such a device have a mass of approximately 20 g, and the therapeutic agent payload is approximately 20 g in mass (60 mm length, allowing room for plug, spring, etc.).

A weight means of mass 81 g is required; which represents a length of 21 mm of iron/steel at the same 25 mm diameter as the dispensing means.

A suitable length for the connecting means is between about 20 and 75 mm, preferably about 50 mm.

2767Y

25430-FF

Example 3

An exemplary device for use in cattle may be sized as follows:

For a device of total length 160 mm and diameter 40 mm, and having a final density of 2.2 $g \cdot mL^{-1}$; the volume of the device is 210 mL, and its mass 440 g.

Plastic components for such a device have a mass of approximately 60 g, and the therapeutic agent payload is approximately 100 g in mass (90 mm length, allowing room for plug, spring, etc.).

A weight means of mass 280 g is required; which represents a length of 28 mm of iron/steel at the same 40 mm diameter as the dispensing means.

A suitable length for the connecting means is between about 25 and 100 mm, preferably about 75 mm.

Although this invention has been described and illustrated in detail with respect to certain preferred embodiments thereof, the scope of the invention is not to be so limited, but is limited only by the scope of the claims (and their equivalents) which follow.

Claims

1. An intraruminal controlled-release device for dispensing a therapeutic agent to the rumen of a ruminant animal, which device comprises:
   dispensing means for dispensing the therapeutic agent, the dispensing means being of lower density than the ruminal content of the animal;
   weight means separate from the dispensing means; and flexible connecting means for connecting the dispensing means to the weight means;
   wherein the weight means is of sufficient size and density to locate at the bottom of the rumen and, with the connecting means, hold the dispensing means at least partially within the ruminal contents.

2. The device of claim 1 wherein the device density is at least 1.5 $g \cdot mL^{-1}$, preferably at least 2 $g \cdot mL^{-1}$, preferably at least 2.2 $g \cdot mL^{-1}$.

3. The device of claim 1 or 2 wherein the density of the weight means is between 6 and 10 $g \cdot mL^{-1}$.

4. The device of any of claims 1 to 3 wherein the weight means comprises a metal or metal alloy, said metal or metal alloy preferably being coated with an inert material.

5. The device of any of claims 1 to 4 wherein the connecting means is of sufficient length to allow the dispensing means to adopt an orientation in the rumen independent of the orientation of the weight means.

6. The device of any of claims 1 to 5 wherein the connecting means is of such length as to hold the dispensing means completely within the ruminal contents.

7. The device of any of claims 1 to 6 wherein the dispensing means and the weight means are cylindrical.

8. The device of claim 7 wherein the length of the connecting means is between 0.5 and 5 times the diameter of the cylinder, preferably between 1 and 3 times the diameter of the cylinder, preferably about 2 times the diameter of the cylinder.

9. The device of any of claims 1 to 8 wherein the connecting means comprises a filament of a polymeric material.

10. The device of any of claims 1 to 9 for administration to cattle which has a maximum size of 180 mm in length, 40 mm in diameter.

11. The device of any of claims 1 to 9 for administration to calves which has a maximum size of 120 mm in length, 30 mm in diameter.

12. The device of any of claims 1 to 9 for administration to sheep or goats which has a maximum size of 110 mm in length, 20 mm in diameter.

13. The device of any of claims 1 to 12 which further comprises therapeutic agent loaded within the dispensing means.

14. The device of claim 13 wherein the therapeutic agent comprises an anthelmintic.

15. The device of claim 14 wherein the therapeutic agent comprises oxfendazole.

0 239 039

*FIG.__1.*

*FIG.__2.*

*FIG.__3.*